(19) Europäisches Patentamt
European Patent Office
Office européen des brevets

(11) **EP 1 519 710 B1**

(12) **EUROPEAN PATENT SPECIFICATION**

(45) Date of publication and mention
of the grant of the patent:
**08.08.2007 Bulletin 2007/32**

(51) Int Cl.:
*A61K 8/02* (2006.01)          *A61K 8/30* (2006.01)
*A61Q 19/10* (2006.01)

(21) Application number: **03724485.2**

(22) Date of filing: **06.05.2003**

(86) International application number:
**PCT/US2003/014226**

(87) International publication number:
**WO 2004/004679 (15.01.2004 Gazette 2004/03)**

(54) **SKIN CLEANSING PRODUCTS INCORPORATING CATIONIC COMPOUNDS**

HAUTREINIGUNGSMITTEL ENTHALTEND KATIONISCHE VERBINDUNGEN

PRODUITS NETTOYANTS POUR LA PEAU CONTENANT DES COMPOSES CATIONIQUES

(84) Designated Contracting States:
**DE GB IT**

(30) Priority: **09.07.2002 US 394646 P**
**26.12.2002 US 330458**

(43) Date of publication of application:
**06.04.2005 Bulletin 2005/14**

(73) Proprietor: **KIMBERLY-CLARK WORLDWIDE, INC.**
**Neenah, Wisconsin 54956 (US)**

(72) Inventors:
• **KOENIG, David, W.**
**Menasha, WI 54952 (US)**
• **OTTS, David, R.**
**Appleton, WI 54915 (US)**
• **GOULET, Mike, T.**
**Neenah, WI 54956 (US)**

(74) Representative: **Beacham, Annabel Rose et al**
**Frank B. Dehn & Co.**
**St Bride's House**
**10 Salisbury Square**
**London EC4Y 8JD (GB)**

(56) References cited:
EP-A- 0 688 901          WO-A-00/57843
WO-A-00/61107          WO-A-01/35905
WO-A-01/83665          WO-A-02/49604
US-B1- 6 280 757

**Description**

CROSS-REFERENCE TO RELATED APPLICATIONS

This application claims priority from U.S. Provisional Patent Application No. 60/394,646 filed on July 9, 2002.

BACKGROUND OF THE INVENTION

The present invention relates to skin cleansing products. More particularly, the present invention relates to skin cleansing products which are highly effective in binding and removing from the surface of skin a broad range of harmful microorganisms such as fungi, yeasts, molds, protozoan, and viruses, as well as fecal soil material and other contaminants. The skin cleansing products of the present invention, which may include numerous products such as dry wipes, wet wipes, feminine napkins, facial tissue, bath tissue, etc., incorporate increased concentrations of cationic compounds, such as, for example, octadecyldimethyltrimethoxysilylpropylammonium chloride, which have an effective charge density of from about 0.1 microequivalents/g to about 8000 microequivalents/g, or more which electrically alter the fibers comprising the product. When the fibers of the cleansing product impregnated with the cationic compound contact the skin, the cationic compound contained on and/or within the product binds the contaminants onto the cationic particles and product such that the contaminant may be removed from the skin. This provides a significant advantage in that the contaminant material is not simply dislodged from the skin surface, but is dislodged and bound simultaneously and, therefore, is no longer available for dispersion and future contamination of other areas.

The skin is the largest organ of the human body. As a boundary layer, the skin performs several major functions: it maintains the body at a correct temperature, holds in essential fluids, and protects against toxic agents, microorganisms, and the sun's potentially harmful rays. Proper skin maintenance is essential for good health. For most people, proper skin maintenance begins with daily cleansing.

Human skin is exposed to various contaminants everyday through both contact with various biological fluids such as urine and feces, as well as contact with numerous environmental factors. Examples of contaminants that the skin contacts everyday include yeast, fungi, mold, protozoan and viruses. Although most microbes are negatively charged due to their chemistry and structures, they can adhere to skin, which is also typically negatively charged, through various interactions such as electrostatic interactions, hydrophobic interactions and ligand interactions. Although these attachment mechanisms are not completely understood, their cumulative effect can tightly bind numerous microbes such as *Candida albicans* to skin resulting in inflammation or irritation. Because these contaminants bind to skin through multiple attachment mechanisms, a complex removal strategy may be required to remove the contaminants without damaging the skin.

The above-listed contaminants, as well as numerous others, are often irritating to the skin and can initiate an elaborate cascade of immunological events upon contact with viable skin cells. Ultimately, these events may lead to severe skin irritation, inflamation, and even infection. Skin cleaning on a daily basis can prevent or minimize skin irritation and inflammation caused by the immunological events.

Conventionally, cleaning of the skin has included any activity that kills, binds and/or removes contaminants present on the skin's surface. Traditionally, skin cleaning has been accomplished through the use of compositions such as solution based skin cleaning products, bath tissues, facial tissues, wet wipes, and dry wipes. Wet wipes are especially preferred by many for cleaning of urine and fecal material from babies or elderly adults and typically remove soils through surfactant interaction with the soil. Most wet wipes commercially available contain microbiocidal agents which are typically highly effective against numerous microbes and soil. In contrast, bath or facial tissue, both typically dry applications, clean soil from skin as a result of shear force, affinity, or a combination of both.

WO 00/61107 describes antimicrobial wipes comprising an antimicrobial active, an anionic surfactant, and a proton donating agent.

Microbiocidal agents contained in many cleaning products may, however, irritate the skin of some users due to the potentially harsh chemicals utilized to provide the antimicrobial effect. As such, although wet wipes are generally effective in cleaning and maintaining healthy skin, some wet wipes may be unsuitable for use by some people. Some wet wipes utilized contain harsh surfactants and/or alcohol or other additives which, while effective against numerous microbes, may dry out or chafe skin. Further, use of microbiocidal agents contained in many wet wipes around open wounds is generally discouraged as the killed microbes may cause further infection if they enter the open wounds. Therefore, a need exists in the art for alternative cleaning methods that do not irritate or chafe the skin of the user, yet provide significant cleaning. Further, a need exists for alternative cleaning methods that simply remove microbes and soil from the skin surface without killing the microbe and risking further infection of open wounds.

## SUMMARY OF THE INVENTION

The present invention provides products which can bind and remove various skin contaminant from the skin. The cleansing products of the present invention are highly effective in dislodging and binding numerous contaminants including fungi, yeasts, molds, protozoan, viruses, soils, and other substances from the skin's surface. Significantly, the products of the present invention do not necessarily kill microbes on the skin's surface during removal, but dislodge and bind the microbes through electrostatic interactions between the product and the microbe. It has been discovered that by providing a cleansing substrate comprising a sufficient amount of cationic compounds having an effective charge density of from about 0.1 microequivalents/g to about 8000 microequivalents/g or more and selected from octadecyldimethyltrimethonysilylpropylammonium chloride and 1-methyl-2-Noroleyl-3-oleyl-amidoethyl imidazoline methylsulfate, the fibers comprising the product can be electrically altered such that the resulting product has a Positive Charge Index as defined herein of at least about 52. Such a Positive Charge Index allows numerous types of microbes and contaminants to be electrostatically dislodged from the skin surface, captured and carried away. The cationic compound-containing skin cleansing products of the present invention are safe for use on the skin and in and around wounds, as microbes are removed from the wound surface without a substantial risk of rupturing, and thus the risk of introduction of byproducts from the microbe into wounds is minimized or eliminated.

Briefly, therefore, the present invention is directed to a cleansing product. The product comprises a substrate carrying a cationic compound capable of binding contaminants located on the surface of skin and selected from octadecyldimethyltrimethonysilylpropylammonium chloride and 1-methyl-2-Noroleyl-3-oleyl-amidoethyl imidazoline methylsulfate. The cationic compound has an effective charge density of from about 0.1 microequivalents/g to about 8000 microequivalents/g and the product has a Positive Charge Index of at least about 52.

The present invention is further directed to a cleansing product comprising a substrate carrying a cationic compound capable of binding contaminants located on the surface of skin and selected from octadecyldimethyltrimethonysilylpropylammonium chloride and 1-methyl-2-Noroleyl-3-oleyl-amidoethyl imidazoline methylsulfate. The cationic compound has an effective charge density of from about 500 microequivalents/g to about 8000 microequivalents/g and the product has a Positive Charge Index of at least about 52.

The present invention is further directed to a cleansing product comprising a substrate carrying a cationic compound capable of binding contaminants located on the skin and selected from the group consisting of octadecyldimethyltrimethoxysilylpropylammonium chloride and 1-methyl-2-Noroleyl-3-oleyl-amidoethyl imidazoline methylsulfate. The cationic compound has an effective charge density of from about 1000 microequivalents/g to about 8000 microequivalents/g and the product has a Positive Charge Index of at least about 52.

The present invention is further directed to a cleansing product comprising a woven web material and a cationic compound capable of binding contaminants located on the surface of skin and selected from the group consisting of octadecyldimethyltrimethoxysilylpropylammonium chloride and 1-methyl-2-Noroleyl-3-oleyl-amidoethyl imidazoline methylsulfate. The cationic compound has an effective charge density of from about 1000 microequivalents/g to about 8000 microequivalents/g and the product has a Positive Charge Index of at least about 52.

The present invention is further directed to a cleansing product comprising a non-woven web material and a cationic compound capable of binding contaminants located on the surface of skin and selected from the group consisting of octadecyldimethyltrimethoxysilylpropylammonium chloride and 1-methyl-2-Noroleyl-3-oleyl-amidoethyl imidazoline methylsulfate. The cationic compound has an effective charge density of from about 1000 microequivalents/g to about 8000 microequivalents/g and the product has a Positive Charge Index of at least about 52.

Other features and advantages of this invention will be in part apparent and in part pointed out hereinafter.

## DEFINITIONS

Within the context of this specification, each term or phrase below will include, but not be limited to, the following meaning or meanings:

(a) "Bonded" refers to the joining, adhering, connecting, attaching, or the like, of two elements. Two elements will be considered to be bonded together when they are bonded directly to one another or indirectly to one another, such as when each is directly bonded to intermediate elements.
(b) "Film" refers to a thermoplastic film made using a film extrusion and/or foaming process, such as a cast film or blown film extrusion process. The term includes apertured films, slit films, and other porous films which constitute liquid transfer films, as well as films which do not transfer liquid.
(c) "Layer" when used in the singular can have the dual meaning of a single element or a plurality of elements.
(d) "Meltblown" refers to fibers formed by extruding a molten thermoplastic material through a plurality of fine, usually circular, die capillaries as molten threads or filaments into converging high velocity heated gas (e.g., air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly dispersed meltblown fibers. Such a process is disclosed for example, in U.S. Patent 3,849,241 to Butin et al. Meltblown fibers are microfibers which may be continuous or discontinuous, are generally smaller than about 0.6 denier, and are generally self bonding when deposited onto a collecting surface. Meltblown fibers used in the present invention are preferably substantially continuous in length.

(e) "Nonwoven" refers to materials and webs of material which are formed without the aid of a textile weaving or knitting process.

(f) "Polymeric" includes, but is not limited to, homopolymers, copolymers, such as for example, block, graft, random and alternating copolymers, terpolymers, etc. and blends and modifications thereof. Furthermore, unless otherwise specifically limited, the term "polymeric" shall include all possible geometrical configurations of the material. These configurations include, but are not limited to, isotactic, syndiotactic and atactic symmetries.

(g) "Positive Charge Index" refers to the amount of positive charge contained on the surface of a substrate as measured by a Positive Charge Index Assay.

(h) "Positive Charge Index Assay" refers to an eosinol assay which utilizes Eosin Y or Bosin B as a biological stain to measure the Positive Charge Index of a substrate.

(i) "Thermoplastic" describes a material that softens when exposed to heat and which substantially returns to a non-softened condition when cooled to room temperature.

DETAILED DESCRIPTION OF THE PREFERRED EMBODIMENTS

In accordance with the present invention, it has been discovered that numerous microbes and soils such as, for example, *Candida albicans,* attached to the skin can be effectively dislodged, captured and removed away from the skin's surface through the use of a cleansing product or substrate comprising a suitable amount of cationic compounds, such as, for example, octadecyldimethyltrimethoxysilylpropylammmonium chloride, having a suitable effective charge density or anion exchange capacity which modifies the overall charge density of the product. Surprisingly, the cleansing products of the present invention are highly effective in removing microbes and soil from the skin, yet are very gentle and non-irritating. Advantageously, the cleansing products of the present invention do not necessarily kill cells or puncture cell walls during skin cleansing, but simply dislodge and bind the contaminant from the skin surface allowing its removal. By facilitating the release and binding of skin contaminants, the products of the present invention significantly improve wound healing and skin health without being substantially irritating to the wound and/or surrounding skin.

The cationic compounds described herein can be incorporated into or.onto a substrate or product utilizing numerous methods. In one embodiment of the present invention, the cationic compounds are impregnated into the fibers comprising the underlying substrate of the cleansing product during the substrate manufacturing process. Although generally referred to herein as "pulp fibers" or "cellulose fibers," it should be recognized that various types of fibers, including wood pulp fibers and synthetic and polymer-type fibers, are suitable for substrate use in the cleansing products of the present invention, and are within the scope of the present invention. Suitable substrates for incorporation of the cationic compounds include, for example, cellulosic materials, coform materials, woven webs, non-woven webs, spunbonded fabrics, meltblown fabrics, knit fabrics, wet laid fabrics, needle punched webs, or combinations thereof.

The cationic compounds contained in the products of the present invention appear to electrostatically interact with contaminants such as fungi, yeasts, molds, protozoan, viruses, and other soils and inorganic particles and dislodge the contaminant from the skin's surface and bind the contaminant such that it may be carried away from the skin. As used herein, the term "contaminant" should be read to include Gram negative and Gram positive bacteria, fungi, yeast, molds, protozoan, viruses, fecal material, urine, blood, as well as other soils and organic and inorganic materials.

The cationic compounds impregnated into or onto the products of the present invention do not necessarily kill or inhibit the growth of microbes, but displace and bind the predominantly negatively charged microbes or other contaminants from the wound surface through positive-negative or negative-positive electrostatic interactions. This is highly advantageous in that the cleansing products of the present invention do not require an antimicrobial, bactericidal or bacteriostatic ingredient to be highly effective in safely cleaning skin. When the cleansing products of the present invention are utilized in or around skin wounds, microbes are not simply punctured, killed and left in the wound, but are actually bound to the cationic compounds in or on the fibers of the product and removed from the skin. This may significantly reduce the chance of further infection in and around the wound. Further, the cationic compounds used in the products of the present invention are substantially non-toxic and non-irritating to the wound and surrounding skin.

Without being bound to a particular theory, it appears that by increasing the attractive forces between the cleansing product containing the cationic compounds and the microbe and/or contaminant on or near the skin or wound surface in excess of the forces attracting the microbe and/or contaminant to the skin, cleaning of the skin can be significantly enhanced by dislodging and binding the contaminant to the cationic species added to the product. It appears that the cationic compounds interact with the overall net negative charge of the microbe and/or contaminant causing the detachment of the microbe and/or contaminant from the skin through an electrostatic interaction. The interaction between the cationic compounds and the microbe and/or contaminant appears to be stronger than the combined forces of adhesion that retain the microbe and/or contaminant on or near the skin including hydrophobic interactions, electrostatic interactions, and ligand interactions. Because the microbe and/or contaminant is released from the skin and bound to the charge modified cleansing product, it may be easily and efficiently carried away by the product. This is highly advantageous over more traditional cleaning products as the contaminant is not merely dislodged from the skin or wound surface, but is dislodged and then removed from the surface through interactions with the substrate containing the cationic compounds. A suitable amount of cationic compounds are added to the products of the present invention such that the forces binding the contaminant to the skin surface, such as hydrophobic interactions, electrostatic interactions, and ligand interactions, can be overcome by the attraction to the cationic species.

An important novel aspect of the present invention is that the charge modified cleansing products of the present invention significantly improve skin cleanliness and health without necessarily killing microorganisms present on the surface of the skin. As mentioned above, this can be a critical factor when products are utilized around wounds. Typically, when microorganisms are killed by antimicrobial or bactericidal agents, which are common in commercially available wet wipes, for example, the outer wall of the microorganism is penetrated and opened to allow access by a killing agent such as, for example, an organic acid. Although this typically results in a kill of the microorganism, the inside contents of the microorganism can "spill out" into an open wound and lead to further complications or increased infections. This significant problem is minimized or eliminated by the present invention which releases the microorganism or other contaminant from the skin or wound surface such that it can be transferred to a substrate surface and carried away. The interaction between microorganisms or other contaminants and the charge altered cleansing products of the present invention results in an actual energy transfer, i.e., energy is released and recaptured in the dislodging and rebinding of contaminants from the skin surface to the cleaning substrate. This cleaning mechanism may also be important for the control of certain other skin problems, such as diaper rash.

The cationic compounds of the present invention utilized to increase the overall cationic charge of a product can be easily incorporated into facial tissue, bath tissue, or other substrates during the manufacturing process. During the manufacture of facial tissue, bath tissue, and other paper products, physical and/or optical properties of the product are often altered by the addition of chemical additives. Generally, chemical additives such as softeners, colorants, brighteners, and strength agents are added to the fiber slurry upstream of the headbox in a paper making machine during the manufacturing or converting stages of production to impart certain attributes to the finished product. These chemical additives are typically mixed in a stock chest or stock line where the fiber slurry has a fiber consistency of from about 0.015 to about 5 percent.

To improve the adsorption of wet end chemical additives, the chemical additives are often modified with functional groups to impart an electrical charge when in water. The electrokinetic attraction between positively charged chemical additives and the anionically charged fiber surfaces aids in the deposition and retention of the chemical additives onto the fibers. The amount of the chemical additive that can ultimately be adsorbed or retained in the paper machine wet end generally follows an adsorption curve exhibiting diminishing incremental adsorption with increasing concentration. As a result, the adsorption of water soluble or water dispersible chemical additives may be significantly less than 100 percent, particularly when trying to achieve high chemical additive loading levels.

In the alternative, the chemical additives mentioned above may be applied onto pulp fiber surfaces in the initial or primary pulp processing, providing more consistent chemical additive additions to the pulp fiber and a reduction or elimination of unretained chemical additives in the process water on a paper machine. With this method, the chemical treatment of the pulp fibers may occur prior to, during, or after the drying phase of the pulp processing. The generally accepted methods of drying include flash drying, can drying, flack drying, through air drying, Infrared drying, fluidized bed drying, or any method known in the art. The addition of cationic compounds to increase the overall cationic charge of the finished product in accordance with the present invention may also be applied to wet lap pulp processes without the use of dryers.

The method for applying the cationic additives of the present invention to the pulp fibers may be used in a wide variety of pulp finishing processing, including dry lap pulp, wet lap pulp, crumb pulp, and flash dried pulp operations. By way of illustration, various pulp finishing processes (also referred to as pulp processing) are disclosed in Pulp and Paper Manufacturing: The Pulping of Wood, 2nd Ed., Volume 1, Chapter 12 (Ronald G. MacDonald, Editor). Various methods may be used to apply the cationic compounds described herein to achieve the desired Positive Charge Index including, but not limited to, direct addition to a fiber slurry, spraying, coating, foaming, printing, size pressing, or any other method known in the art. Further, in situations where additional chemical additives other than the cationic compounds of the present invention are to be employed, the chemical additives may be added to the fibrous web in sequence to reduce interactions between the chemical additives.

Typically, bleached-chemical virgin pulp fiber used in the manufacture of paper products such as facial tissue and bath tissue has a low initial Positive Charge Index when introduced into the manufacturing process, and hence has an overall negative charge. Other types of virgin pulp fiber, such as unbleached-chemical fiber, which may have an even lower initial Positive Charge Index may also be used in accordance with the present invention, but are typically less preferred. As discussed above, during processing numerous chemical additives, most of which are cationic in nature, such as softeners, are added to improve the overall characteristics of the finished product. The total addition of cationic compounds to pulp during the conventional manufacturing of skin cleansing products may typically result in a slightly cationically charged finished product. Such a conventional finished product may have a Positive Charge Index of no more than about 50.

In accordance with the present invention, an amount of cationic compounds in excess of the amounts typically used in the manufacturing process of skin cleansing products is added to the pulp during or after manufacturing to alter the electric charge of the cellulose fibers comprising the product from negative to positive (or from very slightly positive to more positive) to increase the Positive Charge Index of the finished skin cleansing product such that the product retains a strongly positive surface charge. Such a surface charge makes the skin cleansing product highly effective in binding and removing contaminants from the skin's surface through electrostatic interactions during use.

As noted above, the Positive Charge Index of a skin cleansing product is measured in accordance with the present invention utilizing a Positive Charge Index Assay. The Positive Charge Index Assay can utilize Eosin Y or Eosin B as noted below as the reagent. The Positive Charge Index Assay is set forth below.

Positive Charge Index Assay For Determining the Positive Charge Index of a Substrate

The amount of positive charge imparted onto a substrate, such as a base sheet or woven or non-woven web, for example, can be measured in accordance with the present invention using the Positive Charge Index Assay including an anionic dye binding assay. The Positive Charge Index Assay utilizes the dye Eosin Y, which is a biological stain for alkaline materials. Eosin B can optionally be utilized in place of Eosin Y. The Positive Charge Index Assay is carried out as follows:

Step 1: Cut the substrate to be evaluated into two squares approximately 2 centimeters by 2 centimeters. The first square will be stained with Eosin Y as described herein and optically evaluated. The second square will be subjected to the same Eosin Y staining procedure described herein with the exception that the second square will not be stained with Eosin Y; that is, the second square will undergo each and every step as the first square, except Steps 5 and 6 below.

Step 2: Introduce filter paper, such a Whatman #4 Qualitative 125 millimeter filter paper or equivalent, into a Buchner Funnel attached to a vacuum source.

Step 3: Start the vacuum, and wash the filter paper with deionized water.

Step 4: Allow the filter paper to dry.

Step 5: Place the test substrate on top of the dry filter paper and saturate the substrate with 0.75 milliliters of 0.5% (weight/volume) Eosin Y prepared in deionized water.

Step 6: Allow the test substrate to soak in the Eosin Y for 2 minutes and then cover the test substrate with a dry piece of filter paper.

Step 7: Wash the test substrate through the filter paper for 3 minutes with deionized water.

Step 8: Remove the test substrate with forcepts and place it on a dry piece of filter paper and allow it to dry completely.

Step 9: Measure CIELAB Color Space of the dried test substrate using a Minolta CM-508d Spectrophotometer, or similar equipment. The spectrophotometer is set for CIELAB Color Space with the following parameters: Target Status CREEMM, Color Mode L*a*b*, Observer 10°, and the primary Illuminant D65. A standard white block supplied by the spectrophotometer manufacturer is utilized for calibration of the instrument.

Step 10: Calculate the DE*ab value of the Eosin Y stained test substrate using an un-stained test substrate for comparison. The DE*ab value is equal to the Positive Charge Index. The higher the Positive Charge Index, the higher the positive charge on the substrate. The CIE Color System Values are set forth below:

L* = Lightness = A value 0 to 100
a* = Color coordinate red-verses-green
b* = Color coordinate yellow-verses-blue

$$C = \text{Chroma} = [(a*)^2 + (b*)^2]^{1/2}$$

$$h = \text{Hue angle} = \arctan (b*/a*)$$

$$E = \text{Color difference} = [(L*)^2 + (a*)^2 + (b*)^2]^{1/2}$$

$$DL* = L*_{\text{Eosin Stained Substrate}} - L*_{\text{Unstained Substrate}}$$

$$Da* = a*_{\text{Eosin Stained Substrate}} - a*_{\text{Unstained Substrate}}$$

$$Db* = b*_{\text{Eosin Stained Substrate}} - b*_{\text{Unstained Substrate}}$$

$$DE*ab = [(DL*)^2 + (Da*)^2 + (Db*)^2]^{1/2}$$

The cationic compounds useful in the present invention to increase the overall effective cationic charge density of a finished product can easily be incorporated into various skin cleansing products. As used herein, the term "cationic compound" means octadecyldimethyltrimethoxysilylpropylammonium chloride, or 1-methyl-2-Noroleyl-3-oleyl-amidoethyl imidazoline methylsulfate and mixtures and combinations thereof.

The cationic compounds for incorporation into the skin cleansing products of the present invention have a net cationic charge, and may sometimes be referred to as anion exchangers. Typically, the products of the present invention contain cationic compounds having sufficient positive charge to impart improved cleaning characteristics into the products through electrostatic interactions with microbes and/or contaminants and skin. The amount of "cationic charge" on a particular compound can vary substantially and can be measured utilizing several different units. Anionic exchangers are sometimes referred to as having a "capacity" which may be measured in microequivalents per gram or milliequivalents per gram, or may be measured in terms of the amount of a certain compound or protein that the anionic exchanger will bind. Still another way of referring to the amount of positive charge is in terms of micro or milliequivalents per unit area. One skilled in the art will recognize that the exchange capacity units can be converted from one form to another to calculate proper amounts of anion exchanger for use in the present invention.

In accordance with the present invention, the chemical additives utilized to increase the overall effective cationic charge density of the resulting product have a cationic charge. Cationic compounds useful in the present invention typically have an effective charge density of from about 0.1 microequivalents/g to about 8000 microequivalents/g, more preferably from about 100 microequivalents/g to about 8000 microequivalents/g, still more preferably from about 500 microequivalents/g to about 8000 microequivalents/g, and most preferably from about 1000 microequivalents/g to about 8000 microequivalents/g. As the effective charge density of the cationic material increases, the amount of cationic material required to be added to the pulp manufacturing process typically decreases. Generally, from about 0.01% (by weight of the substrate) to about 25% (by weight of the substrate), preferably from about 0.01% (by weight of the substrate) to about 10% (by weight of the substrate) of cationic material having the above-described effective charge density will be sufficient to increase the overall cationic charge of the resulting product sufficiently for purposes of the present invention. The actual amount of cationic material required for introduction into the pulp manufacturing process may be influenced by numerous other factors including, for example, the amount of steric hindrance in the pulp fibers due to other additives present in the pulp fiber environment, the accessibility of the charges on the pulp fibers, competitive reactions by cationic materials for anionic sites, the potential for multilayer adsorption into the pulp fiber, and the potential for precipitation of anionic materials out of solution.

Without being bound to a particular theory, it is believed that the cationic molecules (which may sometimes also be referred to as "softeners" or "debonders") suitable for use in accordance with the present invention have a cationic charge by virtue of a quaternary nitrogen moiety. During the manufacturing of the skin cleansing product, this cationic charge may be used to attract the cationic molecule to the fiber surface, which is typically anionic in nature. The cationic compounds suitable for use in the present invention may have hydrophobic side chains which impart hydrophobicity to the molecule, making these molecules substantially non-water soluble. As such, these cationic compounds are believed to actually exist in solution as micelles of cationic compound molecules, where the hydrophobic tails are in the interior of the micelle and the cationic charges are exposed to the water phase. When a micelle cluster is adsorbed onto the fiber, more than one molecule is present on the surface, thus creating a site on the fiber with an excess of cationic charge. Once dried, these cationic molecules are likely associated with a counterion (although it may be possible that some are present without counterions which may create a static cationic charge) to form a net neutral charge. When the treated substrate comes into contact with an aqueous media such as the urine or feces, the counterion is free to dissociate and thus leaves the fiber cationically charged in the region with adsorbed cationic molecules. The cationic charge on the surface of the substrate is then able to attract and retain various microbes and/or contaminants which typically have a negatively charged outer surface.

In one embodiment of the present invention, the cationic compounds of the present invention can be incorporated into substrates for a wet wipe, hand wipe, face wipe, cosmetic wipe, household wipe, hospital wipe, industrial wipe and the like having improved contaminant removing characteristics while being gentle to the skin. Materials suitable for the substrate of the wipe are well known to those skilled in the art, and are typically made from a fibrous wheet material which may be either woven or nonwoven. For example, wet wipe substrates incorporating the cationic compounds of the present invention may include nonwoven fibrous sheet materials which include meltblown, coform, air-laid, bonded-carded web materials, hydroentangled materials, and combinations thereof. Such materials can be comprised of synthetic or natural fibers, or a combination thereof. Typically, wet wipes define a basis weight of from about 25 to about 120 grams per square meter and desirably from about 40 to about 90 grams per square meter.

In a particular embodiment, the wet wipes incorporating the charge modified formulations of the present invention comprise a coform basesheet of polymeric microfibers and cellulosic fibers having a basis weight of from about 60 to about 80 grams per square meter and desirably about 75 grams per square meter. Such coform basesheets are manufactured generally as described in U.S. Patent No. 4,100,324. Typically, such coform basesheets comprise a gas-formed matrix of thermoplastic polymeric meltblown microfibers, such as, for example, polypropylene microfibers, and cellulosic fibers, such as, for example, wood pulp fibers.

The relative percentages of the polymeric microfibers and cellulosic fibers in the coform basesheet can vary over a wide range depending upon the desired characteristics of the wet wipes. For example, the coform basesheet may comprise from about 20 to about 100 weight percent, desirably from about 20 to about 60 weight percent, and more desirably from about 30 to about 40 weight percent of the polymeric microfibers based on the dry weight of the coform basesheet being used to provide the wet wipes.

Alternatively, wet wipe substrates incorporating the cationic compounds of the present invention can comprise a composite which includes multiple layers of materials. For example, the wet wipes may include a three layer composite which includes an elastomeric film or meltblown layer between two coform layers as described above. In such a configuration, the coform layers may define a basis weight of from about 15 to about 30 grams per square meter and the elastomeric layer may include a film material such as a polyethylene metallocene film.

In another embodiment, the cationic compounds of the present invention can be incorporated into a substrate which can be a woven web, non-woven web, spunbonded fabric, meltblown fabric, knit fabric, wet laid fabric, needle punched web, cellulosic material or web, and combinations thereof, for example, to create products such as facial tissue, bathroom tissue, diapers, feminine care product such as sanitary napkins, hand towels, surgical drapes, wound dressings, gowns, bedsheets, pillowcases and the like. Many of these products are utilized to absorb liquids such as urine, feces, menses, and blood which may contain potentially harmful contaminants.

The addition of the cationic compounds to the substrate may be performed using a liquid application treater such as a DAHLGREN® LAS. This application system applies a wet solution comprising the cationic compounds to the substrate followed by a drying process to produce a dry substrate containing the cationic compounds. This system is commercially available and well known to those skilled in the art.

In another embodiment, the cationic compounds can be added to a substrate through spray coating, slot coating and printing, or a combination thereof. With spray coating, the cationic compounds are first thoroughly mixed with a soluble adhesive agent to disperse the cationic compounds throughout the adhesive material. The adhesive material utilized should be substantially soluble in mucus, feces, urine, or water, depending upon the intended application of the resulting product. Further, the adhesive material should be substantially non-reactive with the cationic compounds and should not substantially alter the electrical properties and charges of the particles.

The adhesive material can comprise a soluble adhesive which will partially or completely dissolve upon use of the resulting product in a hydrous environment. Suitable soluble adhesives may include, for example, polyvinyl pyrrolidone and polyvinyl alcohol, and combinations thereof. After the adhesive and cationic compounds are thoroughly mixed, they can be applied onto the desired area of the product of the present invention by spray coating, knifing, or roller coating, for example, and allowed to dry prior to use.

Similar to spray coating, the cationic compounds may be introduced onto substrates through slot coating. In slot coating, an adhesive-cationic compound mixture as discussed above is introduced directly onto the desired area of the pad in "slots" or discrete row patterns.

The products described herein having an increased effective cationic charge density are highly effective in binding and removing microbes and/or certain contaminants from a wound's surface and surrounding skin. Although not required, the products described herein can be used in combination with other additives to further increase the efficacy of the product under certain circumstances. For example, the products described herein can be used in combination with antimicrobial agents, detergents, microbiocides, colorants, or other additives or skin sensitizing chemicals.

EXAMPLE 1

In Part 1 of this Example, several bath tissues including both commercially available bath tissues, and modified bath tissues, along with other non-cellulosic cleaning sheets were evaluated to determine their Positive Charge Index utilizing a Positive Charge Index Assay. In Part 2 of this Example, three of the tested bath tissues and two cleaning sheets were evaluated for their efficacy in removing *Candida albicans* from a surface.

Part 1:

The following samples (test substrates) were evaluated to determine their Positive Charge Index: (1) Scott® commercial bath tissue; (2) Charmin® commercial bath tissue; (3) Northern® commercial bath tissue; (4) Cottonelle® bath tissue without any cationic softener added; (5) Cottonelle® commercial bath tissue (9.4 kilogram/metric ton of cationic softener added); (6) Cottonelle® bath tissue with 15 kilogram/metric ton of cationic softener added; (7) DSX basesheet without the addition of any SILGARD (octadecyldimethyltrimethoxysilylpropyl-ammonium chloride) ; and (8) DSX basesheet with the addition of 0.7% (by weight) SILGARD.

The cationic softener added to the Cottonelle® bath tissues (test substrate numbers 5 and 6) was 1-methyl-2-Noroleyl-3-oleyl amidoethyl imidazolinium methosulfate obtained from Goldschntidt Ag (Hopewell, Virginia) which has an effective charge density (measured) of about 1300 microequivalents/gram. The cationic softener levels referred to above (9.4 kilogram/metric ton and 15 kilogram/metric ton) are the cationic softener additive loadings based on the outer layer furnish in the sheet. The outer two layers (of a three layer sheet) made up about 65% of the total sheet weight. The remaining about 35% by weight in the center of the product had no cationic softener added to it. As such, the levels of cationic softener based on the total sheet weight are 9.4 x 0.65, or 6.1 kilogram/metric ton and 15 x 0.65, or 9.75 kilogram/metric ton based on the total weight of the three layer sheet. The DSX basesheet was a non-cellulosic, non-woven hydroentangled polyester cleaning basesheet.

The Positive Charge Index Assay was done as follows:

Step 1: Each test substrate was cut into two approximate 2 centimeter by 2 centimeter squares. One of the two squares was evaluated optically without any Eosin Y staining, and the other was subjected to the Eosin Y staining as described herein prior to optical evaluation. The test substrate not subjected to the Eosin Y staining was still subjected to each and every other Step of the procedure.

Step 2: A Whatman #4 Qualitative 125 millimeter filter paper (Whatman, Maidstone, England) was introduced into a Buchner Funnel attached to a vacuum source.

Step 3: The vacuum source was activated and the filter paper was washed with deionized water. After washing, the vacuum source was turned off and the filter paper allowed to air dry.

Step 4: The test substrate was introduced onto the dried filter paper in the Buchner Funnel and saturated with 0.75 milliliters of 0.5% (w/v) Eosin Y (Sigma Chemical Company, St. Louis Missouri) prepared in deionized water and allowed to soak for 2 minutes.

Step 5: After soaking, the test substrate was covered with a dry Whatman #4 Qualitative 125 millimeter filter paper and the test substrate washed through the filter paper with deionized water for 3 minutes.

Step 6: After washing, the test substrate was removed with a forceps and placed on dry Whatman #4 Qualitative 125 millimeter filter paper and allowed to air dry for approximately 15 minutes.

Step 7: After drying, the CIELAB Color Space of the test substrate was measured using a Minolta CM-508d Spectrophotometer (Minolta, Japan). Standard White (Minolta, Japan) was utilized as the calibration color. The spectrophotometer was set for CIELAB Color Space with the following parameters: Target Status CREEMM, Color Mode L\*a\*b\*, Observer 10°, and the primary illuminant D65.

Step 8: The DE\*ab value of the Eosin Y stained test substrate was calculated utilizing the corresponding unstained test substrate for comparison. The DE\*ab value was equal to the Positive Charge Index for the substrate. The higher the Positive Charge Index, the higher the charge on the surface of the substrate. The following CIE Color System Values were utilized:

L\* = Lightness = A value 0 to 100
a\* = Color coordinate red-verses-green
b\* = Color coordinate yellow-verses-blue

$$C = \text{Chroma} = [(a*)^2 + (b*)^2]^{1/2}$$

$$h = \text{Hue angle} = \arctan(b*/a*)$$

$$E = \text{Color difference} = [(L*)^2 + (a*)^2 + (b*)^2]^{1/2}$$

$$DL* = L*_{\text{Eosin Stained Substrate}} - L*_{\text{Unstained Substrate}}$$

$$Da* = a*_{\text{Eosin Stained Substrate}} - a*_{\text{Unstained Substrate}}$$

$$Db* = b*_{\text{Eosin Stained Substrate}} - b*_{\text{Unstained Substrate}}$$

$$DE*ab = [(DL*)^2 + (Da*)^2 + (Db*)^2]^{1/2}$$

The raw data collected on the test substrates is set forth in Table 1, and calculated values are set forth in Table 2.

Table 1: CIELAB Color Space Raw Data

| Sample | L* | a* | b* | C | h |
|---|---|---|---|---|---|
| Standard White | 96.003 | 0.004 | -0.008 | 0.009 | 295.282 |
| Unstained Substrate | | | | | |
| Scott® Commercial | 97.603 | -0.056 | 0.579 | 0.582 | 95.541 |
| Charmin® Commercial | 97.517 | -0.253 | 1.362 | 1.368 | 100.516 |
| Northern® Commercial | 96.962 | -0.346 | 2.088 | 2.117 | 99.411 |
| Cottonelle® (0 kg/mt cationic softener*) | 97.399 | -0.120 | 0.897 | 0.905 | 97.608 |
| Cottonelle® Commercial (9.4 kg/mt cationic softener) | 97.529 | -0.157 | 1.021 | 1.033 | 98.740 |
| Cottonelle® (15 kg/mt cationic softener) | 97.433 | -0.164 | 1.042 | 1.055 | 98.952 |
| DSX Basesheet (0% SILGARD) | 97.020 | 0.019 | 0.284 | 0.285 | 86.128 |
| DSX Basesheet (0.7% SILGARD) | 97.502 | 0.546 | -3.549 | 3.591 | 278.747 |
| Eosin Y Stained Substrate | | | | | |
| Scott® Commercial | 89.491 | 20.500 | -6.179 | 21.411 | 343.228 |
| Charmin® Commercial | 78.279 | 41.203 | -12.813 | 43.150 | 342.725 |
| Northern® Commercial | 83.664 | 32.199 | -8.910 | 33.409 | 344.532 |
| Cottonelle® (0 kg/mt cationic softener) | 89.708 | 17.303 | -5.429 | 18.135 | 342.580 |
| Cottonelle® Commercial (9.4 kg/mt cationic softener) | 77.911 | 43.881 | -14.171 | 46.112 | 342.103 |
| Cottonelle® (15 kg/mt cationic softener) | -25.383 | 56.057 | -17.227 | 57.134 | 63.902 |
| DSX Basesheet (0% SILGARD) | -4.172 | 3.776 | 1.512 | 3.914 | 5.826 |
| DSX Basesheet (0.7% SILGARD) | -24.406 | 46.832 | -6.262 | 44.792 | 53.180 |

Table 2: Calculated Data: CIELAB Color Space Data Relative To Unstained Test Substrate

| Sample | DL* | Da* | Db* | DC | DE*ab |
|---|---|---|---|---|---|
| Cottonelle® (0 kg/mt cationic softener) | -7.691 | 17.423 | -6.326 | 17.231 | 20.068 |
| Scott® Commercial | -8.112 | 20.556 | -6.757 | 20.830 | 23.109 |
| Northern® Commercial | -13.298 | 32.545 | -10.999 | 31.292 | 36.838 |
| Charmin® Commercial | -19.239 | 41.456 | -14.176 | 41.764 | 47.850 |

(continued)

| Sample | DL* | Da* | Db* | DC | DE*ab |
|---|---|---|---|---|---|
| Cottonelle® Commercial (9.4 kg/mt cationic softener) | -19.618 | 44.038 | -15.192 | 45.079 | 50.547 |
| Cottonelle® (15 kg/mt cationic softener) | -25.383 | 56.057 | -17.227 | 57.134 | 63.902 |
| DSX Basesheet (0% SILGARD) | -4.172 | 3.776 | 1.512 | 3.914 | 5.826 |
| DSX Basesheet (7% SILGARD) | -24.406 | 46.832 | -6.262 | 44.792 | 53.180 |

Part 2:

Three separate bath tissues and two non-cellulosic, non-woven hydroentangled polyester cleaning basesheets were evaluated for their ability to remove *Candida albicans* from a skin tape strip. The bath tissues evaluated included: (1) Cottonelle® (0 kg/mt of cationic softener); (2) Cottonelle® Commercial (9.4 kg/mt of cationic softener); and (3) Cottonelle® (15 kg/mt of cationic softener). The cleaning basesheets included a non-cellulosic, non-woven hydroentangled polyester sheet (DSX) without the addition of any SILGARD and a non-cellulosic, non-woven hydroentangled polyester sheet (DSX) with the addition of 0.7% (by weight) SILGARD.

*Candida albicans* (ATCC 10231) was obtained from the American Type Culture Collection (ATCC) (Rockville, Maryland) and was subcultured for two days prior to experimentation onto a Sabourands medium fortified with glucose (SAB-Dex) agar plate (Becton Dickinson, Cockeysville, Maryland) overnight at 37°C. The following day, 2-3 isolated *Candida albicans* colonies were inoculated into SAB-Dex Broth (20 mL) and incubated for 18 hours at 32°C while shaking at 220 rpm. The resulting broth culture was diluted to $1 \times 10^5$ CFU/mL with phosphate buffer (pH = 7.2) (VWR Industries, Batavia Illinois).

The following procedure was utilized to determine the ability of each bath tissue to remove *Candida albicans* from skin tape strips. Skin tape strips were made by pulling D-Squame skin sampling disks (CuDerm Corporation, Dallas, Texas) four times from adjacent adult volar forearm sites. The skin tape strips were then placed into deep six-well plates (Becton Dickinson, Franklin Lakes, New Jersey). The skin tape strips were then blocked with 2 mL of 5% Bovine Serum Albumin (BSA) (Sigma, St. Louis, Missouri) in Phosphate Buffer Solution (150 mM NaCl, 50 mM Potassium Phosphate at a pH = 7.4) for 60 minutes and shaken at 220 rpm at 33°C. After shaking, each well's fluid was removed and 1mL of $10^5$ CFU/mL *Candida albicans* was added to each tape strip. After the addition of the *Candida albicans,* 1 mL of Trypticase Soy Broth (Difco Labs, Detroit, Michigan) was added to each skin tape strip and the plates incubated at 33°C while shaking at 220 rpm for 60 minutes. After shaking, the fluid was aspirated away and the skin tape strips were washed 3 times with 3 mL Tris-Buffered Saline (50 mM Tris (base), 150 mM NaCl at a pH = 7.5). After washing, each skin tape strip was placed in a new 6-well plate and 0.5 mL of Tris Buffered Saline was added to each well.

After the skin tape strips were prepared, blotters, for the determination of the affinity of each bath tissue for the *Candida albicans,* were prepared. Blotters were prepared by placing a new D-Squame skin sampling disc over the end of a 15 x 45 mm borosilicate glass open top screw cap vial (Kimble Glass Inc, VWR, Chicago, Illinois) and placing the bath tissue over the sampling disc and securing it with tape. Three separate blotters were prepared, one for each test substrate. No blotter was utilized on the control skin tape strip.

Each blotter was placed over a designated skin tape strip for 3 minutes. At the beginning of the three minute period, a firm push was exerted on the bottom side of the blotter for about 1 second. After the three minutes, each blotter was removed and discarded. The fluid remaining in each well was aspirated off and each skin tape strip was washed 3 times with 3 mL of Tris Buffered Phosphate. After this washing, each skin tape strip was ready for analysis for the removal of *Candida albicans.*

Each skin tape strip, including the control, was fixed by adding 2 mL of 2.5% Glutaraldehyde to each well and allowing it to stand for 10 minutes. Then each skin tape strip was then washed 3 times with 3 mL of deionized water and then 1.0 mL of 1N sodium hydroxide was added to each skin tape strip. Excess sodium hydroxide was aspirated off the tape strip to produce a moist skin tape strip. Each skin tape strip was then stained by adding 0.5 mL Calcfluor White (Difco, Ann Arbor, Michigan) to the wells for about 5 minutes after which the skin tape strips were washed 3 times with 3 mL deionized water.

Once the skin tape strips were air-dried the *Candida albicans* cells were enumerated visually with a fluorescent microscope. The skin tape strips were placed with the white crescent label near the bottom edge onto a microscope slide perpendicular to the microscope objective. A 20 X objective was employed so that the field of view dissected the skin tape strip in the middle. Only the cells in this middle field of view (an area of about $2 \times 10^7$ micrometers$^2$) were counted. The field of view was about 5% of the total tape strip. The percent removal of *Candida albicans* from the skin tape strips was calculated according to the following formula:

$$[(\text{Control \# of Cells} - \text{Sample \# of Cells})/\text{Control \# of Cells}] \times 100$$

Approximately 5000-10,000 *Candida albicans* cells bound to a 22 mm diameter D-Squame skin tape strip under the conditions of this experiment.
Table 3 shows the results of Part 2 of this Example:

Table 3

| Test Bath Tissue | Positive Charge Index | Cleaning Efficacy (% Removal) |
|---|---|---|
| Cottonelle® (0 kg/mt cationic softener) | 20.1 | 35 |
| Cottonelle® Commercial (9.4 kg/mt cationic softener) | 50.5 | 62 |
| Cottonelle® (15 kg/mt cationic softener) | 63.9 | 81 |
| DSX Sheet (0% SILGARD) | 5.8 | 48.8 |
| DSX Sheet (0.7% SILGARD) | 53.2 | 77.2 |

As the cleaning efficacy data indicate, as the Positive Charge Index of the test substrate increases, its ability to remove *Candida albicans* from the skin tape also increases leading to a higher percentage removal.
In view of the above, it will be seen that the several objects of the invention are achieved. As various changes could be made in the above-described wound management products without departing from the scope of the invention, it is intended that all matter contained in the above description be interpreted as illustrative and not in a limiting sense.

**Claims**

1.  A cleansing product comprising a substrate carrying from about 0.01% (by weight of the substrate) to about 10% (by weight of the substrate) of a cationic compound capable of binding contaminants located on skin, said cationic compound having an effective charge density of from about 0.1 microequivalents/g to about 8000 microequivalents/g, a positive charge imparting a Positive Charge Index of at least about 52 to said product, and being selected from the group consisting of octadecyldimethyltrimethoxysilylpropylammonium chloride, 1-methyl-2-Noroleyl-3-oleyl-amidoethyl imidazoline methylsulfate, and mixtures and combinations thereof.

2.  The product as set forth in claim 1 wherein the substrate is selected from the group consisting of coform materials, woven webs, non-woven webs, spunbonded fabrics, meltblown fabrics, wet-laid fabrics, needle punched webs, cellulosic material, and mixtures and combinations thereof.

3.  The product as set forth in claim 2 wherein the product is selected from the group consisting of facial tissue, bathroom tissue, diapers, sanitary napkins, hand towels, surgical drapes, wound dressings, gowns, bedsheets, pillowcases, dry wipes, and wet wipe substrates.

4.  The product as set forth in any one of claims 1 to 3 wherein the substrate comprises from about 0.1% (by weight of the substrate) to about 10% (by weight of the substrate) of the cationic compound.

5.  The product as set forth in any one of claims 1 to 4 wherein the product has a Positive Charge Index of at least about 55.

6.  The product as set forth in any one of claims 1 to 5 wherein the product has a Positive Charge Index of at least about 60.

7.  The product as set forth in any one of claims 1 to 6 wherein the Positive Charge Index of the product is determined by utilizing an anionic dye binding assay.

8.  The product as set forth in claim 7 wherein the Positive Charge Index of the product is determined by utilizing an anionic dye binding assay selected from the group consisting of an Eosin Y anionic dye binding assay and an Eosin B anionic dye binding assay.

9. The product as set forth in claim 7 wherein the Positive Charge Index of the product is determined by utilizing an Eosin Y anionic dye binding assay.

10. The cleansing product as set forth in any of claims 1 to 9, wherein said cationic compound has an effective charge density of from about 500 microequivalents/g to about 8000 microequivalents/g.

11. The cleansing product in any one of claims 1 to 10, wherein said cationic compound has an effective charge density of from about 1000 microequivalents/g to about 8000 microequivalents/g

12. A cleansing product in any one of claims 1 to 11, wherein the substrate comprises a non-woven web material.


**Patentansprüche**

1. Reinigungsprodukt bzw. Reinigungsmittel, das ein Substrat umfasst, das etwa 0,01 bis etwa 10 Gew.-% (bezogen auf das Gewicht des Substrats) einer kationischen Verbindung trägt, die in der Lage ist, Kontaminanten, die auf der Haut angeordnet sind, zu binden, wobei die kationische Verbindung eine wirksame Ladungsdichte von etwa 0,1 bis etwa 8000 Mikroäquivalenten/g und eine positive Ladung, die dem Reinigungsmittel einen positiven Ladungsindex von mindestens etwa 52 verleiht, aufweist und ausgewählt ist aus der Gruppe, die besteht aus Octadecyl-dimethyl-trimethoxysilyl-propylammoniumchlorid, 1-Methyl-2-noroleyl-3-oleyl-amidoethyl-imidazolin-methylsutfat und Mischungen und Kombinationen davon.

2. Produkt nach Anspruch 1, in dem das Substrat ausgewählt ist aus der Gruppe, die besteht aus Coform-Materialien, Stoff-Bahnen, Vliesstoff-Bahnen, Spunbonded-Geweben, Meitblown-Geweben, Wet-Laid-Geweben, vernadelten Bahnen, Cellulosematerial und Mischungen und Kombinationen davon.

3. Produkt nach Anspruch 2, das ausgewählt ist aus der Gruppe, die besteht aus Gesichts-Tissue, Bad-Tissue, Windeln, Damenbinden, Handtüchem, chirurgischen Tüchern, Wundverbänden, Umhängen, Bettlaken, Kissenbezügen, trokkenen Wischtüchern und feuchten Wischtuch-Substraten.

4. Produkt nach einem der Ansprüche 1 bis 3, bei dem das Substrat etwa 0,1 bis etwa 10 Gew.-% (bezogen auf das Gewicht des Substrats) der kationischen Verbindung umfasst.

5. Produkt nach einem der Ansprüche 1 bis 4, das einen positiven Ladungsindex von mindestens etwa 55 aufweist.

6. Produkt nach einem der Ansprüche 1 bis 5, das einen positiven Ladungsindex von mindestens etwa 60 aufweist.

7. Produkt nach einem der Ansprüche 1 bis 6, bei dem der positive Ladungsindex des Produkts bestimmt wird durch Anwendung eines anionischen Farbstoff-Bindungs-Assays.

8. Produkt nach Anspruch 7, bei dem der positive Ladungsindex des Produkts bestimmt wird durch Anwendung eines anionischen Farbstoff-Bindungs-Assays, ausgewählt aus der Gruppe, die besteht aus einem Eosin Y-anionischen Farbstoff-Bindungs-Assay und einem Eosin B-anionischen Farbstoff-Bindungs-Assay.

9. Produkt nach Anspruch 7, bei dem der positive Ladungsindex des Produkts bestimmt wird durch Anwendung eines Eosin Y-anionischen Farbstoff-Bindungs-Assays.

10. Reinigungsprodukt nach einem der Ansprüche 1 bis 9, bei dem die kationische Verbindung eine wirksame Ladungsdichte von etwa 500 bis etwa 8000 Mikroäquivalenten/g aufweist.

11. Reinigungsprodukt nach einem der Ansprüche 1 bis 10, bei dem die kationische Verbindung eine wirksame Ladungsdichte von etwa 1000 bis etwa 8000 Mikroäquivalenten/g aufweist.

12. Reinigungsprodukt nach einem der Ansprüche 1 bis 11, bei dem das Substrat ein Vliesstoff-Bahnmaterial umfasst.

**Revendications**

1. Produit nettoyant comprenant un substrat portant d'environ 0,01 % (en poids du substrat) à environ 10 % (en poids du substrat) d'un composé cationique capable de se lier à des contaminants situés sur la peau, ledit composé cationique présentant une densité de charge effective d'environ 0,1 microéquivalent/g à environ 8000 microéquivalents/g, une charge positive conférant un indice de charge positive d'au moins environ 52 audit produit, et étant choisi parmi le groupe constitué du chlorure d'octadécyldiméthyltriméthoxysilylpropylammonium, du méthylsulfate de 1-méthyl-2-noroléyl-3-oléyl-amidoéthylimidazoline, et de mélanges et de combinaisons de ceux-ci.

2. Produit selon la revendication 1, dans lequel le substrat est choisi parmi le groupe constitué de matières « coform », de nappes tissées, de nappes non tissées, de tissus filés-liés, de tissus obtenus par fusion-soufflage, de tissus obtenus par voie humide, de nappes aiguilletées, d'une matière cellulosique, et de mélanges et de combinaisons de ceux-ci.

3. Produit selon la revendication 2, le produit étant choisi parmi le groupe constitué d'un mouchoir pour le visage, de papier toilette, de couches-culottes, de serviettes hygiéniques, de serviettes de toilette, de champs stériles, de pansements, de blouses, de draps, de taies d'oreiller, d'éponges sèches et de substrats à éponge humide.

4. Produit selon l'une quelconque des revendications 1 à 3, dans lequel le substrat comprend d'environ 0,1 % (en poids du substrat) à environ 10 % (en poids du substrat) du composé cationique.

5. Produit selon l'une quelconque des revendications 1 à 4, le produit présentant un indice de charge positive d'au moins environ 55.

6. Produit selon l'une quelconque des revendications 1 à 5, le produit présentant un indice de charge positive d'au moins environ 60.

7. Produit selon l'une quelconque des revendications 1 à 6, l'indice de charge positive du produit étant déterminé en employant un dosage de liaison à un colorant anionique.

8. Produit selon la revendication 7, l'indice de charge positive du produit étant déterminé en employant un dosage de liaison à un colorant anionique, choisi parmi le groupe constitué d'un dosage de liaison au colorant anionique Eosin Y et d'un dosage de liaison au colorant anionique Eosin B.

9. Produit selon la revendication 7, l'indice de charge positive du produit étant déterminé en employant un dosage de liaison au colorant anionique Eosin Y.

10. Produit nettoyant selon l'une quelconque des revendications 1 à 9, dans lequel ledit composé cationique présente une densité de charge effective d'environ 500 microéquivalents/g à environ 8000 microéquivalents/g.

11. Produit nettoyant selon l'une quelconque des revendications 1 à 10, dans lequel ledit composé cationique présente une densité de charge effective d'environ 1000 microéquivalents/g à environ 8000 microéquivalents/g.

12. Produit nettoyant selon l'une quelconque des revendications 1 à 11, dans lequel le substrat comprend une matière de nappe non tissée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 39464602 P
- WO 0061107 A
- US 3849241 A, Butin
- US 4100324 A

**Non-patent literature cited in the description**

- Pulp and Paper Manufacturing: The Pulping of Wood. vol. 1